# EUROPEAN PATENT APPLICATION

(11) **EP 2 996 057 A1**
(43) Date of publication of application: **16.03.2016**
(21) Application number: 14184618.8
(22) Date of filing: 12.09.2014
(51) Int. Cl.: G06F 19/00

(54) **Healthcare related information management**

(71) Applicant: Oulun Ammattikorkeakoulu Oy, 90220 Oulu (FI)
(72) Inventor: Kangasoja, Jussi, 91900 Liminka (FI); Nisula, Pekka, 90900 Kiiminki (FI); Orajärvi, Kaisa, 91500 Muhos (FI)
(74) Representative: Papula Oy

(57) **Abstract**

The invention allows management of healthcare related information utilizing an apparatus comprising a screen, a communication means, at least one processor, and at least one memory including computer program code. Healthcare related information of a subject human stored in the at least one memory is periodically and automatically synchronized with at least one external database via the communication means. At least a part of a computer generated three-dimensional anatomical model of at least a part of a body of the subject human is displayed on the screen. An input command selecting a location of the displayed at least part of the three-dimensional anatomical model is received. A portion of the healthcare related information of the subject human mapped to the selected location is retrieved from the at least one memory. The retrieved portion of the healthcare related information is displayed on the screen.

## Description

### FIELD OF THE INVENTION

The present invention relates to management of healthcare related information. In particular, the present invention relates to management of healthcare related information utilizing an apparatus comprising a screen and a communication means.

### BACKGROUND OF THE INVENTION

Today, healthcare professionals such as doctors and nurses typically need to access several different medical databases in order to manage healthcare related information of a given patient. For example, one database may contain healthcare records, while another database may contain prescription records.

Typically, these databases need to be accessed manually and one at a time. Also, once a database is accessed, it typically presents all the data records related to a given patient and the user needs to manually search for the relevant portion(s).

All in all, current management systems of healthcare related information are unintuitive, complex and cumbersome to interact with.

Accordingly, an object of the present invention is to alleviate the problems described above and to introduce a solution that allows simple and intuitive management of healthcare related information utilizing an apparatus comprising a screen and a communication means.

### SUMMARY OF THE INVENTION

A first aspect of the present invention is an apparatus that comprises a screen, a communication means, at least one processor and at least one memory including computer program code. The at least one memory and the computer program code are configured to, with the at least one processor, cause the apparatus at least to perform:
synchronizing healthcare related information of a subject human stored in the at least one memory periodically and automatically with at least one external database via the communication means;
displaying, on the screen, at least a part of a computer generated three-dimensional anatomical model of at least a part of a body of the subject human;
receiving an input command selecting a location of the displayed at least part of the three-dimensional anatomical model;
retrieving, from the at least one memory, a portion of the healthcare related information of the subject human mapped to the selected location; and
displaying the retrieved portion of the healthcare related information on the screen.

A second aspect of the present invention is a method of utilizing an apparatus comprising a screen, a communication means, at least one processor, and at least one memory including computer program code. Healthcare related information of a subject human stored in the at least one memory is periodically and automatically synchronized with at least one external database via the communication means. At least a part of a computer generated three-dimensional anatomical model of at least a part of a body of the subject human is displayed on the screen. An input command selecting a location of the displayed at least part of the three-dimensional anatomical model is received. A portion of the healthcare related information of the subject human mapped to the selected location is retrieved from the at least one memory. The retrieved portion of the healthcare related information is displayed on the screen.

A third aspect of the present invention is a computer program that comprises code adapted to cause the following when executed on an apparatus comprising a screen, a communication means, at least one processor, and at least one memory:
synchronizing healthcare related information of a subject human stored in the at least one memory periodically and automatically with at least one external database via the communication means;
displaying, on the screen, at least a part of a computer generated three-dimensional anatomical model of at least a part of a body of the subject human;
receiving an input command selecting a location of the displayed at least part of the three-dimensional anatomical model;
retrieving, from the at least one memory, a portion of the healthcare related information of the subject human mapped to the selected location; and
displaying the retrieved portion of the healthcare related information on the screen.

In an embodiment of the invention, one of the healthcare related information of the subject human and the mapped portion of the healthcare related information is further synchronized with the at least one external database via the communication means in response to receiving the input command.

In an embodiment of the invention, heuristics are utilized to map locations of the computer generated three-dimensional anatomical model and portions of the healthcare related information of the subject human to each other.

In an embodiment of the invention, the healthcare related information is distributed between at least two external databases, and it is determined which of the at least two external databases to synchronize with based on synchronization data stored in the at least one memory.

In an embodiment of the invention, the healthcare related information comprises at least one of alphanumerical data, image data, audio data and video data.

In an embodiment of the invention, the healthcare related information comprises at least one of healthcare records data, prescription records data, and collected health metrics data associated with the subject human.

In an embodiment of the invention, the apparatus is portable, the screen comprises a touch screen, the communication means comprises a wireless communication means, and the input command comprises an input touch command.

It is to be understood that the aspects and embodiments of the invention described above may be used in any combination with each other. Several of the aspects and embodiments may be combined together to form a further embodiment of the invention. An apparatus, a method, or a computer program which is an aspect of the invention may comprise at least one of the embodiments of the invention described above.

The invention allows simple and intuitive management of healthcare related information utilizing an apparatus comprising a screen and a communication means. For example, the invention allows automatic synchronization of healthcare related information with external databases. Furthermore, the invention allows displaying only healthcare related information relevant to a body location of a patient selected by a user rather than all the healthcare related information of the patient stored in a given database. Furthermore, the invention allows displaying simultaneously healthcare related information of a patient retrieved from several databases.

### BRIEF DESCRIPTION OF THE DRAWINGS

The accompanying drawings, which are included to provide a further understanding of the invention and constitute a part of this specification, illustrate embodiments of the invention and together with the description help to explain the principles of the invention. In the drawings:
**Figure 1** is a signaling diagram illustrating a method according to an embodiment of the invention;
**Figure 2** is a block diagram illustrating an apparatus according to an embodiment of the invention; and
**Figures 3a** and **3b** illustrate a graphic user interface displayed on the screen of the apparatus of the invention.

### DETAILED DESCRIPTION OF THE INVENTION

Reference will now be made in detail to the embodiments of the present invention, examples of which are illustrated in the accompanying drawings.

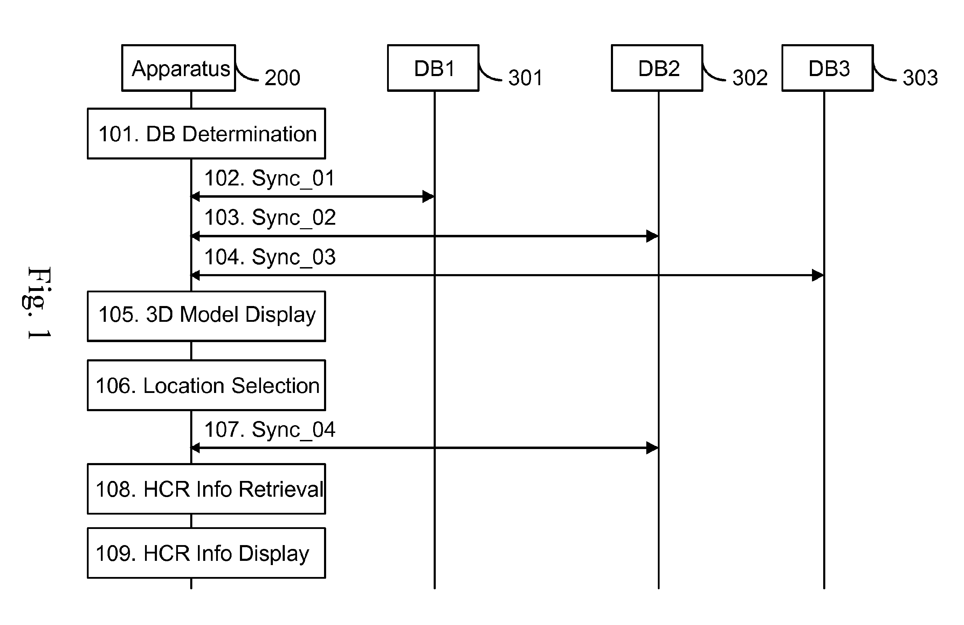

Figure 1 is a signaling diagram illustrating a method of managing healthcare related information according to an embodiment of the present invention.

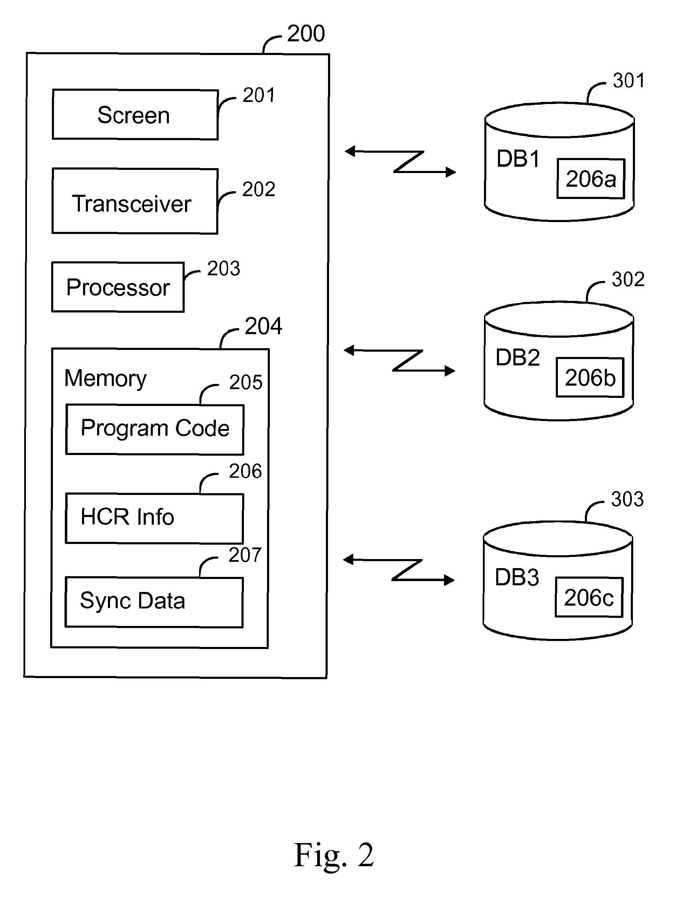

The examples of Figures 1 and 2 utilize a portable apparatus, such as a tablet computer or a smart phone, comprising a touch screen and a wireless communication means. However, the invention is not limited to tablet computers and smart phones. Instead, e.g. a desktop computer or a laptop computer may be utilized.

The method utilizes a portable apparatus 200 comprising a touch screen 201, a wireless communication means 202, at least one processor 203, and at least one memory 204 including computer program code 205. These will be discussed in more detail in connection with Figure 2.

The healthcare related information may comprise e.g. healthcare records data, prescription records data, and/or collected health metrics data associated with a subject human. Furthermore, the healthcare related information may be in the form of e.g. alphanumerical data, image data, audio data and/or video data. User of the portable apparatus 200 of the invention may be e.g. a healthcare professional such as a doctor or a nurse, and the subject human may be e.g. a patient. In another embodiment, the subject human/patient may also be the user of the portable apparatus 200.

In the embodiment shown in Figures 1 and 2, the healthcare related information is distributed between three external databases 301, 302, 303. However, it is to be understood that the invention is not limited to three external databases. Instead, any number of external databases may be utilized. In the embodiment shown in Figures 1 and 2, the external database 301 may comprise e.g. healthcare records data 206a, the external database 302 may comprise e.g. prescription records data 206b, and the external database 303 may comprise e.g. collected health metrics data 206c (such as e.g. blood sugar level, blood pressure, and the like) associated with the subject human.

At step 101, it is determined which one or which ones of the external databases 301-303 to synchronize with based on synchronization data 207 stored in the at least one memory 204. The synchronization data 207 may e.g. contain information on which databases comprises which particular type of healthcare related information (e.g. healthcare records data, prescription records data 206b, or collected health metrics data). Furthermore, the synchronization data 207 may contain information on how to get the healthcare related information from each of the external databases 301-303 (e.g. which interface to use, etc.). For example, an application programming interface (API) block may be generated for at least one of the external databases 301-303. The generated API block may comprise information about at least one of: access rights, format of the data to be retrieved (e.g. image, text, video), identification (e.g. user name, password), and location/address of the data to be retrieved (e.g. an Internet Protocol (IP) address).

Based on the determination performed in step 101, healthcare related information 206 of the subject human stored in the at least one memory 204 is automatically (i.e. without requiring input from the user of the portable apparatus 200) synchronized with the external databases 301-303 via the wireless communication means 202, steps 102, 103, 104. However, it is to be understood that steps 102-104 of Figure 1 are merely an example. Instead, any of the synchronization steps may be performed at any suitable point. Furthermore, the synchronization steps are repeated (not shown in Figure 1) periodically to keep the healthcare related information 206 stored in the at least one memory 204 up-to-date.

At step 105, at least a part of a computer generated three-dimensional (3D) anatomical model of at least a part of a body of the subject human is displayed on the touch screen. Herein, the term "3D model" is used to refer to a model that is generated by a computer as a mathematical representation of a three-dimensional object, i.e. at least a part of a body of the subject human. As is known in the art, the 3D model may be displayed visually as a two-dimensional (2D) image through a process called 3D rendering. It is to be understood that herein the 3D model may not necessarily be an exact representation of the at least part of the body of the subject human. Instead, e.g. a generic 3D model of at least a part of a body of a generic human may be used to which portions of the healthcare related information of the subject human may be mapped.

The displayed 3D model may be manipulated by the user in various ways via touch commands. The user may e.g. pan, zoom in, zoom out, rotate, and select body parts/locations. At step 106, an input touch command selecting a location of the displayed at least part of the 3D anatomical model is received. In other words, the user has selected a body part/location about which he/she wishes to acquire healthcare related information. For example, the user may select the body part/location "head" to view information related to e.g. head and/or mouth of the subject human.

At optional step 107, all of the healthcare related information 206 or a portion of the healthcare related information 206 mapped to the location selected at step 106 may be further synchronized with the respective external database(s) via the wireless communication means 202 in response to receiving the input touch command. The optional step 107 allows the healthcare related information 206 to be as up-to-date as possible since it is synchronized just before the display step 109.

According to the invention, various portions of the healthcare related information 206 of the subject human are mapped to their associated locations/body parts in the 3D model of the at least part of the body of the subject human. Heuristics may be utilized to map locations of the computer generated three-dimensional anatomical model and portions of the healthcare related information of the subject human to each other. For example, the computer generated 3D anatomical model of at least the part of the body of the subject human may be divided into smaller 3D models for e.g. muscles, organs etc. that may be grouped. The location of the displayed 3D model selected with the input command of the user and optionally its associated group may be mapped with the healthcare related information of the subject human related to the selected location and its group. The healthcare related information to be retrieved may be pre-identified or organized to enable this mapping. E.g. an object identifier (OID) used in HL7 7 (Health Level 7) standards for transfer of clinical and administrative data between hospital information systems may be utilized for the identification. Herein, the term heuristics is used to refer to a feature of the invention that enables linking the selected location and its group to other group(s) based on symptoms of the subject human. For example, neck and shoulder area symptoms may be linked to arm/hand motor coordination.

At step 108, the portion of the healthcare related information 206 of the subject human mapped to the selected location is retrieved from the at least one memory 204. At step 109, the retrieved portion of the healthcare related information 206 is displayed on the touch screen 201. The retrieved portion of the healthcare related information 206 may be displayed e.g. close to the selected location of the 3D model.

Figure 2 is a block diagram illustrating a portable apparatus 200 according to an embodiment of the invention. The portable apparatus 200 comprises a touch screen 201, a wireless communication means 202 (e.g. a wireless transceiver), at least one processor 203 and at least one memory 204 including computer program code 205. The portable apparatus 200 may comprise e.g. a tablet computer or a smart phone or the like. The wireless communication means 202 may utilize any suitable wireless communication technology (e.g. wireless local area network (WLAN), and/or cellular mobile telecommunications) to communicate with the external databases 301-303. The communication path may include e.g. a wireless access network and the Internet.

The at least one memory 204 and the computer program code 205 are configured to, with the at least one processor 203, cause the portable apparatus 200 at least to perform:
synchronizing healthcare related information 206 of a subject human stored in the at least one memory 204 periodically and automatically with at least one external database 301-303 via the wireless communication means 202;
displaying, on the touch screen 201, at least a part of a computer generated three-dimensional anatomical model of at least a part of a body of the subject human;
receiving an input touch command selecting a location of the displayed at least part of the three-dimensional anatomical model;
retrieving, from the at least one memory 204, a portion of the healthcare related information 206 of the subject human mapped to the selected location; and
displaying the retrieved portion of the healthcare related information on the touch screen 201.

The at least one memory 204 and the computer program code 205 may be further configured to, with the at least one processor 203, cause the portable apparatus 200 to further synchronize one of the healthcare related information 206 of the subject human and the mapped portion of the healthcare related information 206 with the at least one external database 301-303 via the wireless communication means 202 in response to receiving the input touch command.

The at least one memory 204 and the computer program code 205 may be further configured to, with the at least one processor 203, cause the portable apparatus 200 to utilize heuristics to map locations of the computer generated three-dimensional anatomical model and portions of the healthcare related information 206 of the subject human to each other.

The healthcare related information 206a, 206b, 206c may be distributed between several external databases 301-303, and the at least one memory 204 and the computer program code 205 may be further configured to, with the at least one processor 203, cause the portable apparatus 200 to determine which of the external databases 301-303 to synchronize with based on synchronization data 207 stored in the at least one memory 204.

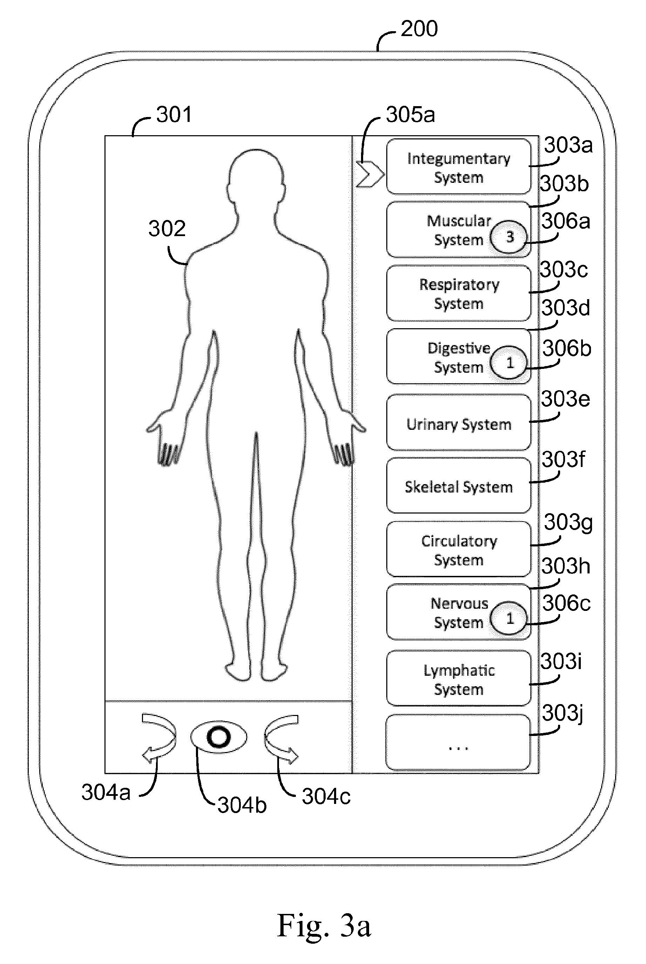

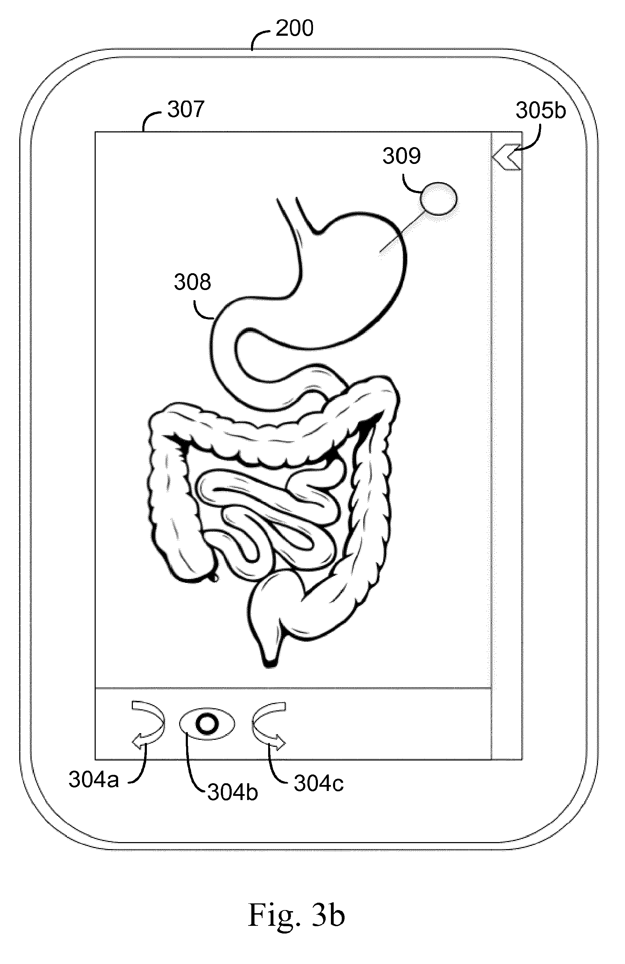

Figures 3a and 3b illustrate a graphic user interface displayed on the screen of the apparatus 200 of the invention. Figure 3a illustrates a main view 301 in which a computer generated three-dimensional anatomical model 302 of a body of a subject human is displayed. Symbols or icons 304a-304c are for manipulating the position and view of the model 302. More specifically, symbol 304a is for rotating the model 302 clockwise, and symbol 304c is for rotating the model 302 counterclockwise. Symbol 304b is for resetting the model 302 back to its initial position after having been rotated with symbols 304a and/or 304c. Buttons 303a-303j enable selecting various subsystem views, such as the digestive system view 307 shown in Figure 3b. Numbers 306a-306c inside the buttons 303a-303j indicate the amount of existing pieces of the healthcare related information associated with the respective subsystem. No number inside a button indicates no existing healthcare related information associated with the respective subsystem.

Figure 3b illustrates a subsystem view. More specifically, Figure 3b illustrates the digestive system view 307 that can be reached by actuating the button 303d in the main view 301 of Figure 3a. The digestive system view 307 displays a computer generated three-dimensional anatomical model 308 of the digestive system of the subject human. Symbols or icons 304a-304c are for manipulating the position and view of the model 308 similarly to Figure 3a. In Figure 3b, the pin 309 indicates the one added piece of the healthcare related information (indicated by the number 306b in Figure 3a) associated with the digestive system. As discussed above, the healthcare related information may comprise e.g. alphanumerical data, image data, audio data and/or video data. Arrow symbols 305a-305b are for hiding/showing the buttons 303a-303j.

It is to be understood that Figures 3a and 3b are merely examples and various other arrangements for the graphic user interface may be implemented.

The exemplary embodiments can include, for example, any suitable desktop computers, laptop computers, tablet computers, smartphones, handheld wireless computing devices, database servers, and the like, capable of performing the processes of the exemplary embodiments. The devices and subsystems of the exemplary embodiments can communicate with each other using any suitable protocol and can be implemented using one or more programmed computer systems or devices.

One or more interface mechanisms can be used with the exemplary embodiments, including, for example, Internet access, telecommunications in any suitable form (e.g., voice, modem, and the like), wireless communications media, and the like. For example, employed communications networks or links can include one or more wireless communications networks, cellular communications networks, 3G communications networks, 4G communications networks, Public Switched Telephone Network (PSTNs), Packet Data Networks (PDNs), the Internet, intranets, a combination thereof, and the like.

It is to be understood that the exemplary embodiments are for exemplary purposes, as many variations of the specific hardware used to implement the exemplary embodiments are possible, as will be appreciated by those skilled in the hardware and/or software art(s). For example, the functionality of one or more of the components of the exemplary embodiments can be implemented via one or more hardware and/or software devices.

The exemplary embodiments can store information relating to various processes described herein. This information can be stored in one or more memories, such as a hard disk, optical disk, magneto-optical disk, RAM, and the like. One or more databases can store the information used to implement the exemplary embodiments of the present inventions. The databases can be organized using data structures (e.g., records, tables, arrays, fields, graphs, trees, lists, and the like) included in one or more memories or storage devices listed herein. The processes described with respect to the exemplary embodiments can include appropriate data structures for storing data collected and/or generated by the processes of the devices and subsystems of the exemplary embodiments in one or more databases.

All or a portion of the exemplary embodiments can be conveniently implemented using one or more general purpose processors, microprocessors, digital signal processors, micro-controllers, and the like, programmed according to the teachings of the exemplary embodiments of the present inventions, as will be appreciated by those skilled in the computer and/or software art(s). Appropriate software can be readily prepared by programmers of ordinary skill based on the teachings of the exemplary embodiments, as will be appreciated by those skilled in the software art. In addition, the exemplary embodiments can be implemented by the preparation of application-specific integrated circuits or by interconnecting an appropriate network of conventional component circuits, as will be appreciated by those skilled in the electrical art(s). Thus, the exemplary embodiments are not limited to any specific combination of hardware and/or software.

Stored on any one or on a combination of computer readable media, the exemplary embodiments of the present inventions can include software for controlling the components of the exemplary embodiments, for driving the components of the exemplary embodiments, for enabling the components of the exemplary embodiments to interact with a human user, and the like. Such software can include, but is not limited to, device drivers, firmware, operating systems, development tools, applications software, and the like. Such computer readable media further can include the computer program product of an embodiment of the present inventions for performing all or a portion (if processing is distributed) of the processing performed in implementing the inventions. Computer code devices of the exemplary embodiments of the present inventions can include any suitable interpretable or executable code mechanism, including but not limited to scripts, interpretable programs, dynamic link libraries (DLLs), Java classes and applets, complete executable programs, Common Passenger Request Broker Architecture (CORBA) passengers, and the like. Moreover, parts of the processing of the exemplary embodiments of the present inventions can be distributed for better performance, reliability, cost, and the like.

As stated above, the components of the exemplary embodiments can include computer readable medium or memories for holding instructions programmed according to the teachings of the present inventions and for holding data structures, tables, records, and/or other data described herein. Computer readable medium can include any suitable medium that participates in providing instructions to a processor for execution. Such a medium can take many forms, including but not limited to, non-volatile media, volatile media, and the like. Non-volatile media can include, for example, optical or magnetic disks, magneto-optical disks, and the like. Volatile media can include dynamic memories, and the like. Common forms of computer-readable media can include, for example, a floppy disk, a flexible disk, hard disk, magnetic tape, any other suitable magnetic medium, a CD-ROM, CD±R, CD±RW, DVD, DVD-RAM, DVD±RW, DVD±R, HD DVD, HD DVD-R, HD DVD-RW, HD DVD-RAM, Blu-ray Disc, any other suitable optical medium, punch cards, paper tape, optical mark sheets, any other suitable physical medium with patterns of holes or other optically recognizable indicia, a RAM, a PROM, an EPROM, a FLASH-EPROM, any other suitable memory chip or cartridge, a carrier wave or or any other suitable medium from which a computer can read.

While the present inventions have been described in connection with a number of exemplary embodiments, and implementations, the present inventions are not so limited, but rather cover various modifications, and equivalent arrangements, which fall within the purview of prospective claims.

## Claims

1. An apparatus (200), comprising:
a screen (201);
a communication means (202);
at least one processor (203); and
at least one memory (204) including computer program code (205),
**characterized in** the at least one memory (204) and the computer program code (205) being configured to, with the at least one processor (203), cause the apparatus (200) at least to perform:
synchronizing healthcare related information (206) of a subject human stored in the at least one memory (204) periodically and automatically with at least one external database (301, 302, 303) via the communication means (202);
displaying, on the screen (201), at least a part of a computer generated three-dimensional anatomical model of at least a part of a body of the subject human;
receiving an input command selecting a location of the displayed at least part of the three-dimensional anatomical model;
retrieving, from the at least one memory (204), a portion of the healthcare related information (206) of the subject human mapped to the selected location; and
displaying the retrieved portion of the healthcare related information (206) on the screen (201).

2. The apparatus (200) according to claim 1, wherein the at least one memory (204) and the computer program code (205) are further configured to, with the at least one processor (203), cause the apparatus (200) to further synchronize one of the healthcare related information (206) of the subject human and the mapped portion of the healthcare related information (206) with the at least one external database (301, 302, 303) via the communication means (202) in response to receiving the input command.

3. The apparatus (200) according to claim 1 or 2, wherein the at least one memory (204) and the computer program code (205) are further configured to, with the at least one processor (203), cause the apparatus (200) to utilize heuristics to map locations of the computer generated three-dimensional anatomical model and portions of the healthcare related information (206) of the subject human to each other.

4. The apparatus (200) according to any of claims 1-3, wherein the healthcare related information (206a, 206b, 206c) is distributed between at least two external databases (301, 302, 303), and wherein the at least one memory (204) and the computer program code (205) are further configured to, with the at least one processor (203), cause the apparatus (200) to determine which of the at least two external databases (301, 302, 303) to synchronize with based on synchronization data (207) stored in the at least one memory (204).

5. The apparatus (200) according to any of claims 1-4, wherein the healthcare related information (206, 206a, 206b, 206c) comprises at least one of alphanumerical data, image data, audio data and video data.

6. The apparatus (200) according to any of claims 1-5, wherein the healthcare related information (206, 206a, 206b, 206c) comprises at least one of healthcare records data, prescription records data, and collected health metrics data associated with the subject human.

7. The apparatus (200) according to any of claims 1-5, wherein the apparatus (200) is portable, the screen (201) comprises a touch screen, the communication means (202) comprises a wireless communication means, and the input command comprises an input touch command.

8. A method of utilizing an apparatus comprising a screen, a communication means, at least one processor, and at least one memory including computer program code,
**characterized in** the method comprising:
synchronizing (102, 103, 104) healthcare related information of a subject human stored in the at least one memory periodically and automatically with at least one external database via the communication means;
displaying (105), on the screen, at least a part of a computer generated three-dimensional anatomical model of at least a part of a body of the subject human;
receiving (106) an input command selecting a location of the displayed at least part of the three-dimensional anatomical model;
retrieving (108), from the at least one memory, a portion of the healthcare related information of the subject human mapped to the selected location; and
displaying (109) the retrieved portion of the healthcare related information on the screen.

9. The method according to claim 8, further comprising further synchronizing (107) one of the healthcare related information of the subject human and the mapped portion of the healthcare related information with the at least one external database via the communication means in response to receiving (106) the input command.

10. The method according to claim 8 or 9, further comprising utilizing heuristics to map locations of the computer generated three-dimensional anatomical model and portions of the healthcare related information of the subject human to each other.

11. The method according to any of claims 8-10, wherein the healthcare related information is distributed between at least two external databases, and further comprising determining (101) which of the at least two external databases to synchronize with based on synchronization data stored in the at least one memory.

12. The method according to any of claims 8-11, wherein the healthcare related information comprises at least one of alphanumerical data, image data, audio data and video data.

13. The method according to any of claims 8-12, wherein the healthcare related information comprises at least one of healthcare records data, prescription records data, and collected health metrics data associated with the subject human.

14. The method according to any of claims 8-13, wherein the apparatus is portable, the screen comprises a touch screen, the communication means comprises a wireless communication means, and the input command comprises an input touch command.

15. A computer program comprising code adapted to cause the following when executed on an apparatus comprising a screen, a communication means, at least one processor, and at least one memory:
synchronizing (102, 103, 104) healthcare related information of a subject human stored in the at least one memory periodically and automatically with at least one external database via the communication means;
displaying (105), on the screen, at least a part of a computer generated three-dimensional anatomical model of at least a part of a body of the subject human;
receiving (106) an input command selecting a location of the displayed at least part of the three-dimensional anatomical model;
retrieving (108), from the at least one memory, a portion of the healthcare related information of the subject human mapped to the selected location; and
displaying (109) the retrieved portion of the healthcare related information on the screen.

16. The computer program according to claim 15, wherein the computer program is stored on a computer readable medium.
